# EUROPEAN PATENT SPECIFICATION

(11) **EP 3 140 040 B1**
(45) Date of publication and mention of the grant of the patent: **06.05.2020**
(21) Application number: 15725519.1
(22) Date of filing: 08.05.2015
(51) Int. Cl.: B01L 3/00

(54) **SAMPLE APPLICATOR FOR POINT OF CARE DEVICE**
PROBENAPPLIKATOR FÜR POINT-OF-CARE-VORRICHTUNG
APPLICATEUR D'ÉCHANTILLONS POUR DISPOSITIF DE POINT DE SOINS

(30) Priority: 08.05.2014 US 201461990611 P; 08.05.2014 EP 14167545
(43) Date of publication of application: 15.03.2017
(73) Proprietor: Radisens Diagnostics Ltd., Co. Cork P31 HF29 (IE)
(72) Inventor: O'BRIEN, Jerry, Co. Cork P31 HF29 (IE); DOOLAN, David, Co. Cork P31 HF29 (IE)
(74) Representative: Purdylucey Intellectual Property
(86) International application number: PCT/EP2015/060216
(87) International publication number: WO 2015/169956

(56) References cited:
- EP-A1- 0 211 334
- JP-A- 2008 032 695
- US-A- 4 740 472
- US-A1- 2009 205 447
- Carl A Burtis ET AL: "Developmentof a Simple Devicefor ProcessingWhole-BloodSamplesinto MeasuredAliquotsof Plasma", CLIN.CHEM. CLINICAL CHEMISTRY, 1 January 1986 (1986-01-01), pages 1642-1647, XP055489263, Retrieved from the Internet: URL:http://clinchem.aaccjnls.org/content/c linchem/32/9/1642.full.pdf [retrieved on 2018-06-29]

## Description

### Field

The disclosure relates to a sample applicator apparatus, system and method, for use in a point of care diagnostic device for application of liquid samples.

### Background

Manual processing to determine the cellular/biological content of various types of biological samples, and in particular samples that contain living cells, is cost-prohibitive in many applications and is also prone to errors. Automation is also cost-prohibitive in many applications, and is inappropriate as currently practiced - using, for example, liquid handling robots - for applications such as point-of-care or doctor's office analysis.

There have been many recent advances in point-of-care diagnostic assay systems based on centrifugal microfluidic technologies. Such systems typically comprise i) a centrifugal microfluidic cartridge with reagent storage and sample processing methods, and ii) related device readers for interrogation of samples processed on such centrifugal microfluidic cartridges. However, there is an unmet need to provide a simple method of biological sample application, compared with current point-of-care centrifugal microfluidic based diagnostic assay systems, that i) is less prone to user error, ii) minimises biohazard and aerosol contamination risk, iii) removes the requirement of cartridge cleaning, iv) simplifies user workflow protocols v) simplifies cartridge manufacture and cost, and vi) integrates user fail-safe mechanisms.

Existing centrifugal-based point-of-care diagnostic assay systems typically use either i) an external transfer pipette for application of liquid samples, or ii) an inlet capillary port integrated on the cartridge, whereby the sample is applied directly onto the cartridge. While the cartridges associated with both system approaches can perform a variety of integrated sample preparation and assay tests - such as lateral flow assays, electrochemical assays, etc. - their sample application methods do not address the aforementioned unmet need.

Consider the first case of an external transfer pipette. In this instance, a biological sample is applied to the transfer pipette through capillary action upon contact by the pipette's tip with the sample. The pipette tip is then typically inserted into the centrifugal cartridge's inlet chamber which is situated close to the cartridge's centre. The sample is dispensed (or transferred), for example, through either an integrated air-displacement piston within the pipette or squeezing of a rubber bulb on top of the pipette, depending on the pipette's design. This sample application method suffers the risk of aerosol or biohazard contamination once the centrifugal cartridge is spun. While integrating an absorbent material into the cartridge's inlet chamber reduces this risk, it does not eliminate it, and further complicates the cartridge's manufacturing process. Covering the inlet chamber with a physical barrier increases cost and biohazard risk, and adds user workflow steps, thereby increasing user training requirements.

Consider the second case of an integrated inlet capillary port. In this instance, a biological sample is applied directly onto a cartridge's inlet capillary port from, for the example of whole blood, a patient's lanced finger. The inlet capillary port typically protrudes somewhat from the cartridge to facilitate both user operation and sample application. Such methods typically require advanced user training as positioning the inlet capillary port to contact the patient's finger can be problematic and lead to unsuccessful or poor quality application. Such integrated inlet capillary ports complicate the cartridge's manufacturing process adding to cost and reducing production yield. They also require the application of a physical barrier, as in the previous case, to minimise biohazard and aerosol contamination.

There are a numerous examples in the art which illustrate the first case. Examples include US4898832 (Boehringer Mannheim), JP 2008 032695 (Matsushita) US5061381 (Abaxis) and US6143248 (Gamera) which describe various sample processing methods, but all use external transfer pipettes to load the sample.

One such example of the second case in the art is US2009/205447 (Panasonic) which describes a system for transferring a sample liquid dispensed as a drop on an inlet port. The inlet port is formed to protrude in a direction away from the chamber, a recessed section is formed around the injection port, and the inlet port is arranged on the side of a rotating axis centre so that centrifugal force, upon its rotation, transfers the sample to said chamber. A hinged cover mechanism prevents biohazard and aerosol contamination.

Other examples of microfluidic systems employing capillaries and centrifugal forces are disclosed in US 4,740,572 (US Energy), EP 0 211 334 (US Energy) and a paper published by Carl A Burtis et. al. published 1 January 1986, pages 1642-1647 Clin. Chem. Clinical Chemistry, entitled ' Development of a Simple Device for Processing Whole Blood Samples into Measured Aliquots of Plasma'.

It is therefore an object to provide a low-cost, simple sample application apparatus and method to address at least one problem known in the art.

### Summary

The invention is defined by the independent claims. Preferred embodiments result from the dependent claims. Any explanation falling outside the scope of the claims is for information only. In one embodiment the force applied to dispense the biological sample from the transfer pipette is a centrifugal force caused by rotation of the cartridge. The fluidic barrier within the transfer pipette prevents the escape of biological sample through the opposite end of the transfer pipette.

In one embodiment the biological sample is replaced with an otherwise constituted sample of liquid form.

In another embodiment there is provided a transfer pipette for use with a microfluidic system to process biological samples comprising:
a channel to collect and store the biological sample; and
an air vent to ensure transfer of air during the biological sample application to, and dispensing from, the transfer pipette to the microfluidic system when a force is applied.

While this invention relates and applies to a myriad biological samples, such as whole blood, saliva, serum, sweat, etc., the specific embodiment described herein concentrates on the sampling of whole blood from a patient's finger.

### Brief Description of the Drawings

The invention will be more clearly understood from the following description of an embodiment thereof, given by way of example only, with reference to the accompanying drawings, in which:-
Figure 1 illustrates a transfer pipette inserted into a receiving chamber of a centrifugal cartridge according to one embodiment of the invention;
Figure 2 illustrates a whole blood sample from a lanced finger being applied to the removed transfer pipette according to one embodiment of the invention; and
Figure 3 illustrates schematically the transfer pipette and disc structures which provide the mechanism to dispense the applied sample into an integrated output chamber.

### Detailed Description of the Invention

Herein is described a sample application method comprising a transfer pipette wherein a sample is applied to same through capillary action, said pipette is inserted into a receiving chamber on a centrifugal cartridge, with the pipette designed to ensure the sample is dispensed into an integrated output chamber.

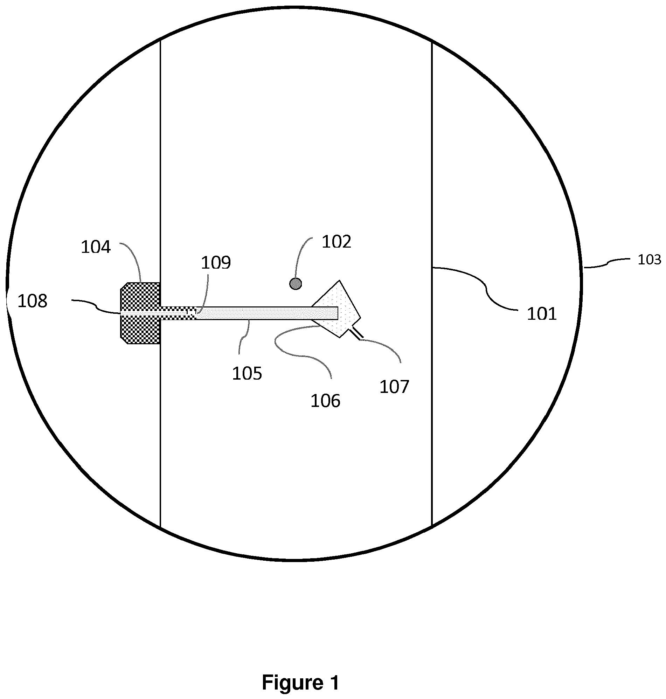

Figure 1 illustrates a high-level sample applicator and cartridge construction comprising a centrifugal microfluidic cartridge 101 with centre 102, which is coupled to a rotary motor (not shown), and circumference 103 drawn with two segments removed, according to one embodiment of the invention. The sample applicator comprises a transfer pipette with handle 104 attached to its sample transfer body 105. Figure 1 illustrates the transfer pipette inserted within the cartridge, without a sample applied. Later illustrations describe geometrical relationships and the function of the integrated output chamber 106 with an outlet channel 107. An air vent 108 in the transfer pipette's handle, enables air displacement within the transfer pipette to allow sample application and dispensing, through capillary and centrifugal forces, respectively. A fluidic barrier 109 minimises the leakage of applied sample to the outside environment

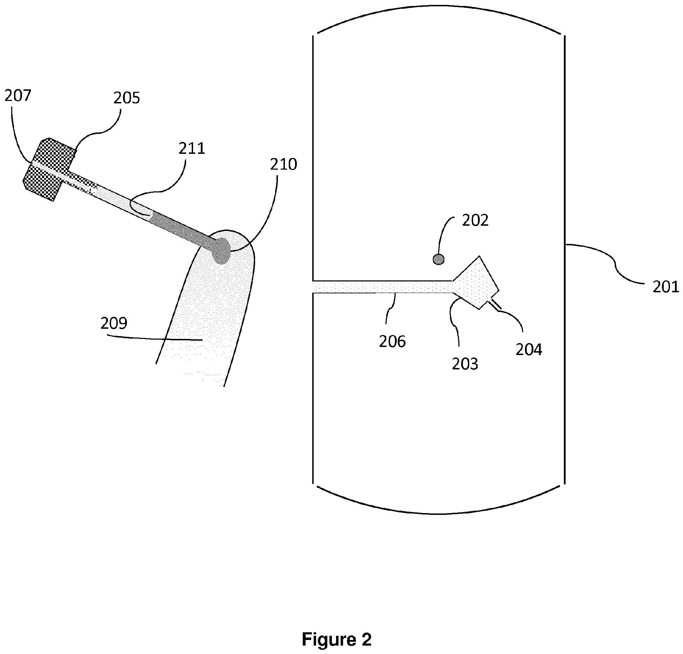

Figure 2 illustrates a typical protocol by which the sample is first applied to the transfer pipette and further details the high-level cartridge structures. Herein is shown a cartridge 201 comprising a centre 202, which is coupled to a rotary motor (not shown), and output chamber 203 with associated outlet channel 204, as already shown in Figure 1. The transfer pipette 205 is removed from the cartridge, thereby showing the receiving chamber 206 on the cartridge, connected to the output chamber which is distal from the receiving chamber. The air vent 207 within the transfer pipette and associated fluidic barrier 208 are as previously described. The figure illustrates the use case for a finger-prick of whole blood, whereby a finger 209 upon being lanced, using a procedure known to those skilled in the art, produces a whole blood sample 210 on the surface of the finger. It will be appreciated that the transfer pipette can receive a sample by any contact with the sample and not necessarily from the surface of the finger. Upon contact with the transfer pipette, the blood sample begins to fill by capillary action towards the pipette head up to a point which shall be defined as the trailing sample meniscus 211.

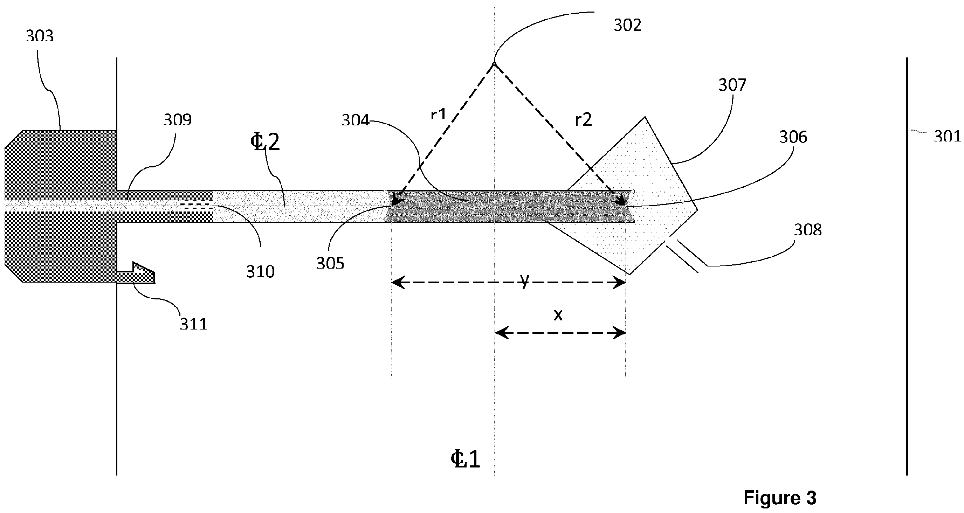

Figure 3 illustrates the detailed workings of this invention. As before, the cartridge 301 comprises centre 302, which is coupled to a rotary motor (not shown). The transfer pipette 303 is re-inserted into the cartridge via the receiving chamber previously illustrated in Figure 2. A first notional centreline 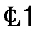 is shown intersecting the cartridge centre and scribed perpendicular to the receiving chamber. A second notional centreline 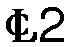 is shown perpendicular to 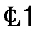 and projects along the centre axis of the transfer pipette. The transfer pipette is shown with applied sample 304 filled between the trailing sample meniscus 305 and leading sample meniscus 306 at the pipette's tip. The radial distance from the cartridge centre to the trailing sample meniscus is noted as r1; the radial distance from the cartridge centre to the leading sample meniscus is noted as r2; and the distance between the leading and trailing menisci is noted as y, which may be described as the approximate height of the applied sample within the transfer pipette. In other words the radial distance of the sample meniscus 305 proximate to the pipette tip should be larger than the radial distance of the sample meniscus 306 distal from the tip. The output chamber 307 is arranged to be distal from the leading sample meniscus. Subsequent sample processing elsewhere in the cartridge may occur through methods and structures connected to the outlet channel 308.

In operation the transfer pipette is designed such that capillary forces retain the sample, unless a pressure is applied to overcome them. When a rotational velocity is applied by the rotary motor to generate a centrifugal force, the net flow of sample in a centrifugal microfluidic cartridge structure will always be radially outwards, i.e. from a proximal radius towards a distal radius. Therefore, once a rotational velocity is applied to generate a centrifugal force greater than the capillary forces at the pipette's tip, the sample will dispense into the distal output chamber, enabled by air displacement through the air vent 309, once the condition r₂>r₁ is maintained. To maintain the relationship, r₂>r₁, the height of the applied sample within the transfer pipette, y, should not exceed two times x (2x), where 2x is defined by mirroring x around 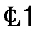 , always noting that 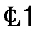 is perpendicular to the receiving chamber. A fluidic barrier 310 is used to minimise the risk of applied sample dispensing into the outside environment prior to application of centrifugal force through the rotary motor's rotation, or otherwise, and is also positioned to ensure y< 2x, by design.

In practice, the aforementioned parameters are dimensioned such that once sufficient centrifugal force is applied to overcome the capillary forces at the tip of the transfer pipette, the sample is dispensed into the more distal output chamber. To avoid inadvertent movement, or removal, of the transfer pipette upon generation of centrifugal force by the rotation of the rotary motor, a single-use locking mechanism 311 may be used, or design variants of same.

## Claims

1. A microfluidic system for processing biological samples comprising:
a transfer capillary pipette (303) comprising an air vent (309) and configured to contain a biological sample (304);
a cartridge (301) adapted to provide at least one receiving chamber which receives said pipette (303), and a distal output chamber wherein the biological sample from the pipette can be dispensed into the for this configured output chamber when
a centrifugal force is applied caused by rotation of the cartridge, **characterised in that** the transfer capillary pipette comprises a fluidic barrier (109, 310) which is configured such that the meniscus of the applicable biological sample (304) at the transfer capillary pipette tip is radially distal from the opposite meniscus within the said transfer pipette and the radial distance (r2) of the biological sample meniscus proximate to the pipette tip from the centre of rotation of the cartridge (302) is larger than the radial distance (r1) of the opposite biological sample meniscus distal from the tip.

2. The microfluidic system as claimed in claim 1 wherein the meniscus of the said applied biological sample (304) at the transfer capillary pipette tip is radially distal from the opposite meniscus within said transfer pipette.

3. The microfluidic system as claimed in claim 1 or 2 wherein the air vent ensures transfer of air during the biological sample application to, and dispensing from, the pipette by capillary action.

4. The microfluidic system as claimed in any preceding claim wherein the said transfer capillary pipette comprises a single-use locking mechanism adapted to prevent removal during rotation by a rotary motor.

5. A method of processing biological samples in a microfluidic system, as claimed in any of claims 1 to 4, said method comprising the steps of:
coupling a transfer pipette to a cartridge; and
rotating the cartridge such that the biological sample from the transfer pipette is dispensed into an output chamber of the cartridge.

## Patentansprüche

1. Mikrofluidisches System zur Verarbeitung biologischer Proben, das Folgendes umfasst:
eine Transferkapillarpipette (303), die eine Lüftungsöffnung (309) hat und so konfiguriert ist, dass sie eine biologische Probe (304) enthält;
eine Kartusche (301), die so ausgestaltet ist, dass sie wenigstens eine Aufnahmekammer zum Aufnehmen der genannten Pipette (303) und eine distale Ausgabekammer bereitstellt, wobei die biologische Probe aus der Pipette in die dafür konfigurierte Ausgabekammer abgegeben werden kann, wenn eine durch Drehen der Kartusche verursachte Zentrifugalkraft aufgebracht wird,
**dadurch gekennzeichnet, dass** die Transferkapillarpipette eine Fluidbarriere (109, 310) umfasst, die so konfiguriert ist, dass der Meniskus der applizierbaren biologischen Probe (304) an der Spitze der Transferkapillarpipette radial distal vom gegenüberliegenden Meniskus innerhalb der genannten Transerpipette ist und der radiale Abstand (r2) des Meniskus der biologischen Probe nahe der Pipettenspitze vom Drehzentrum der Kartusche (302) größer ist als der radiale Abstand (r1) des gegenüberliegenden Meniskus der biologischen Probe distal von der Spitze.

2. Mikrofluidisches System nach Anspruch 1, wobei der Meniskus der genannten applizierten biologischen Probe (304) an der Spitze der Transferkapillarpipette radial distal vom gegenüberliegenden Meniskus innerhalb der genannten Transferpipette ist.

3. Mikrofluidisches System nach Anspruch 1 oder 2, wobei die Lüftungsöffnung den Lufttransfer gewährleistet, während die biologische Probe auf die Pipette durch Kapillarwirkung appliziert oder daraus abgegeben wird.

4. Mikrofluidisches System nach einem vorherigen Anspruch, wobei die genannte Transferkapillarpipette einen Einweg-Verriegelungsmechanismus umfasst, der so ausgestaltet ist, dass er eine Entfernung während der Drehung durch einen Drehmotor verhindert.

5. Verfahren zum Verarbeiten biologischer Proben in einem mikrofluidischen System nach einem der Ansprüche 1 bis 4, wobei das genannte Verfahren die folgenden Schritte beinhaltet:
Koppeln einer Transferpipette mit einer Kartusche; und
Drehen der Kartusche, so dass die biologische Probe aus der Transferpipette in eine Ausgabekammer der Kartusche abgegeben wird.

## Revendications

1. Système microfluidique destiné à traiter des échantillons biologiques comprenant :
une pipette capillaire de transfert (303) comprenant un évent d'air (309) et configurée pour contenir un échantillon biologique (304) ;
une cartouche (301) conçue pour fournir au moins une chambre de réception qui reçoit ladite pipette (303), et une chambre de sortie distale dans lequel l'échantillon biologique de la pipette peut être distribué dans la chambre de sortie configurée pour ceci quand une force centrifuge est appliquée causée par la rotation de la cartouche,
**caractérisé en ce que** la pipette capillaire de transfert comprend une barrière contre les fluides (109, 310) qui est configurée de sorte que le ménisque de l'échantillon biologique applicable (304) au niveau de la pointe de pipette capillaire de transfert est distal de manière radiale par rapport au ménisque opposé au sein de ladite pipette de transfert et la distance radiale (r2) du ménisque d'échantillon biologique à proximité de la pointe de pipette du centre de rotation de la cartouche (302) est supérieure à la distance radiale (r1) du ménisque d'échantillon biologique opposé distal par rapport à la pointe.

2. Système microfluidique selon la revendication 1 dans lequel le ménisque dudit échantillon biologique appliqué (304) au niveau de la pointe de pipette capillaire de transfert est distal de manière radiale par rapport au ménisque opposé au sein de ladite pipette de transfert.

3. Système microfluidique selon la revendication 1 ou 2 dans lequel l'évent d'air assure le transfert d'air pendant l'application de l'échantillon biologique sur la pipette et la distribution à partir de la pipette par action capillaire.

4. Système microfluidique selon l'une quelconque des revendications précédentes dans lequel ladite pipette capillaire de transfert comprend un mécanisme de verrouillage à usage unique conçu pour en empêcher le retrait pendant la rotation par un moteur rotatif.

5. Procédé de traitement d'échantillons biologiques dans un système microfluidique, selon l'une quelconque des revendications 1 à 4, ledit procédé comprenant les étapes de :
couplage d'une pipette de transfert à une cartouche ; et
rotation de la cartouche de sorte que l'échantillon biologique provenant de la pipette de transfert est distribué jusque dans une chambre de sortie de la cartouche.
